# EUROPEAN PATENT APPLICATION

(11) **EP 2 927 850 A1**
(43) Date of publication of application: **07.10.2015**
(21) Application number: 15161651.3
(22) Date of filing: 30.03.2015
(51) Int. Cl.: G06Q 10/06, G06Q 50/22, G06F 19/00

(54) **System and method for the transmission of information about a care-requiring person to caregivers**

(30) Priority: 31.03.2014 JP 2014071010
(71) Applicant: Funai Electric Co., Ltd., Daito-shi Osaka 574-0013 (JP)
(72) Inventor: Babaguchi, Yutaka, Daito-shi, Osaka (JP)
(74) Representative: Grünecker Patent- und Rechtsanwälte PartG mbB

(57) **Abstract**

An information processing apparatus comprising: a receiver that receives data indicating a state of a detection object detected by a state detection unit; and an information transmitter that transmits state information to an external apparatus based on the data, the information transmitter transmitting the state information to at least selected one of: a first external apparatus determined to be at a short distance from the detection object; a second external apparatus determined to have been in proximity to the detection object; and a third external apparatus that is preliminarily determined.

## Description

### BACKGROUND

### (Technical Field)

An aspect of the present invention relates to a care system that detects an abnormality of a care-requiring person in a care facility and that notifies a caregiver of the abnormality.

### (Related Art)

Recently, a care system has been proposed that detects an abnormality of a care-requiring person and that notifies a caregiver of the abnormality. In this type of care system, a sensor installed in a bedside of a care-requiring person is used to detect whether or not any abnormality occurs in the care-requiring person, and upon detection of an abnormality, the occurrence of the abnormality is notified to a mobile terminal of a caregiver who is responsible for this care-requiring person. For example, JP1999-353570A discloses an abnormality detection system that, upon occurrence of an abnormality, transmits an alert signal to a receiver carried by a caregiver.

In such a conventional system, occurrence of an abnormality in a care-requiring person is notified to a caregiver who is responsible for the care-requiring person, which makes it difficult to respond to an emergency if the caregiver who is responsible for the care-requiring person is remote from the care-requiring person.

### SUMMARY

To solve the above-described problems, the present invention adopts the following means. The description given below contains the reference signs on the drawings being placed in parentheses, merely for facilitation of understanding of the invention and not for limiting elements of the present invention. The elements should be construed as widely as within the technical purview of those skilled in the art.

An aspect of the present invention is an information processing apparatus (server 100) including: a receiver that receives data indicating a state of a detection object (care-requiring person 210) detected by a state detection unit (bedside sensor 200); and an information transmitter (wireless transmitter 140) that transmits state information to an external apparatus (mobile terminal 300A, 300B, 300C) based on the data,
the information transmitter transmitting the state information (abnormality information) to at least selected one of:
a first external apparatus (mobile terminal 300B) determined to be at a short distance from the detection object (care-requiring person 210);
a second external apparatus (mobile terminal 300A) determined to have been in proximity to the detection object; and
a third external apparatus (mobile terminal 300C) that is preliminarily determined.

Another aspect of the present invention is an information transmission system including:
a state detection unit (bedside sensor 200) that detects a state of a detection object;
a terminal detection unit (bedside sensor 200) that detects a state of proximity of an external apparatus; and
an information transmitter (the wireless transmitter 140 of the server 100) that, based on a result of detection performed by the state detection unit and a result of detection performed by the terminal detection unit, transmits state information (abnormality information) to at least selected one of:
   a first external apparatus (mobile terminal 300B) determined to be at a short distance from the detection object;
   a second external apparatus (mobile terminal 300A) determined to have been in proximity to the detection object; and
   a third external apparatus (mobile terminal 300C) that is preliminarily determined.

Another aspect of the present invention is a method for transmitting information, including:
a first step of detecting a state of a detection object;
a second step of detecting a state of proximity of an external apparatus; and
a third step of, based on results of detection performed in the first and second steps, transmitting state information to at least selected one of:
   a first external apparatus determined to be at a short distance from the detection object;
   a second external apparatus determined to have been in proximity to the detection object; and
   a third external apparatus that is preliminarily determined.

Another aspect of the present invention is an electronic apparatus including:
a receiver that receives a state information transmitted based on data indicating a state of a detection object; and
a display unit that displays a predetermined image based on the state information,
the receiver receiving the state information that is transmitted to at least selected one of:
   a first external apparatus determined to be at a short distance from the detection object;
   a second external apparatus determined to have been in proximity to the detection object; and
   a third external apparatus that is preliminarily determined.

The information processing apparatus, the information transmission system, the method for transmitting information, or the electronic apparatus having the above configuration can provide an environment that allows an appropriate treatment to be given to the detection object. In particular, application of such an information processing apparatus, and the like, to a care system enables occurrence of a specified state (occurrence of an abnormality) to be notified to one or more persons from: (1) a caregiver B (310B) who possesses the mobile terminal (300B) and exists near the detection object (care-requiring person 210) having the specified state (having the abnormality); (2) a caregiver A (310A) who possesses the mobile terminal (300A) and existed near the detection object (care-requiring person 210) immediately before occurrence of the above-mentioned state; and (3) a caregiver who is responsible for the detection object (care-requiring person 210) having the above-mentioned state. As a result, the caregiver B is able to respond to an emergency, while the caregiver A who is likely to recognize the condition of the detection object (care-requiring person 210) and the responsible caregiver who is likely to familiar with a history of the detection object (care-requiring person 210) are able to give an appropriate treatment. Here, it is preferable that occurrence of the specified state to be notified to more than two persons. According to this configuration, it is possible to give a more appropriate treatment. And it is preferable that the third external apparatus is preliminary associated with the detection object. According to this configuration, it is possible to give a more appropriate treatment.

Preferably, the information processing apparatus or the information transmission system is configured such that
the first external apparatus is determined based on the detection of the state of the detection object,
the second external apparatus is determined based on a situation where the second external apparatus has been in proximity to the detection object prior to the first external apparatus.

In the information processing apparatus or the information transmission system having the above configuration, the first external apparatus is determined based on the detection of the state of the detection object (care-requiring person). This enables the state information to be transmitted in accordance with a change in the state of the detection object. In particular, application of such an information processing apparatus, and the like, to a care system makes it possible that, for example, a caregiver who possesses an external apparatus recognizes a change in the state of the detection object (care-requiring person) more appropriately. In addition, since the second external apparatus is determined based on its proximity to the detection object prior to the first external apparatus, the state information can be transmitted to a person (caregiver A) possessing the second external apparatus, who is likely to know a previous situation of the detection object.

Preferably, the information processing apparatus or the information transmission system is configured such that
the information transmitter (wireless transmitter 140) transmits the state information to the third external apparatus and to at least one of the first and second external apparatuses.

The information processing apparatus or the information transmission system having the above configuration, in which the state information is transmitted to the third external apparatus, enables the state information to be transmitted to a responsible person (responsible caregiver) associated with the detection object (care-requiring person) without fail.

Preferably, the information processing apparatus is configured such that
the first and second external apparatuses to which the state information is to be transmitted are determined based on an input received from a terminal detection unit (bedside sensor 200) that detects a state of proximity of an external apparatus.

The information processing apparatus having the above configuration, in which the state of proximity of the external apparatus is detected based on an input received from the terminal detection unit (bedside sensor 200), is able to determine proximity of the external apparatus more appropriately.

Preferably, the information processing apparatus or the information transmission system is configured such that
in a case where, after the state information is transmitted to the at least one external apparatuses, the terminal detection unit (bedside sensor 200) detects proximity of one of the at least one external apparatuses and then the proximity becomes undetectable,
the information transmitter (wireless transmitter 140) transmits completion information (treatment completion information) to the other of the at least one external apparatuses.

The information processing apparatus or the information transmission system having the above configuration is able to automatically notify a person who possesses another external apparatus of the fact that the treatment of the detection object is completed. More specifically, the above-described information processing apparatus or the like is able to automatically notify a caregiver who does not know the status of the treatment of the abnormality of the fact that the treatment of the abnormality of the care-requiring person is completed.

Preferably, the information processing apparatus or the information transmission system further includes a detection terminal storage unit (memory 130) that stores a result of detection performed by the terminal detection unit (bedside sensor 200) and a detection time associated with the result of detection.

The information processing apparatus or the information transmission system having the above configuration makes it possible that, for example, the server 100 stores the status of detection of an external apparatus (the mobile terminal 300A or 300B) detected by the bedside sensor 200. Accordingly, a process of notifying occurrence of an abnormality can be readily executed.

Preferably, the information processing apparatus or the information transmission system is configured such that:
in a case where, after the state information (abnormality information) is transmitted to the at least one external apparatuses, first response information (treatment declaration information) is received from one of the at least one external apparatuses,
the information transmitter (wireless transmitter 140) transmits first others' response information (others' treatment declaration information) to the other of the at least one external apparatuses.

In the information processing apparatus or the information transmission system having the above configuration, for example, any caregiver who possesses an external apparatus is able to easily notify that he/she is going to treat the abnormality, to a caregiver who possesses another external apparatus.

Preferably, the information processing apparatus or the information transmission system is configured such that:
in a case where, after the state information (abnormality information) is transmitted to the at least one external apparatuses, proximity of one of the at least one external apparatuses is detected,
the information transmitter (wireless transmitter 140) transmits others' proximity information to the other of the at least one external apparatuses.

In the information processing apparatus or the information transmission system having the above configuration, for example, any caregiver who possesses an external apparatus is able to easily notify that he/she has approached the care-requiring person having the abnormality, to a caregiver who possesses another external apparatus. Accordingly, the caregiver who possesses another external apparatus is able to readily recognize that another caregiver is going to treat the abnormality.

Preferably, the information processing apparatus or the information transmission system is configured such that:
in a case where, after the state information (abnormality information) is transmitted to the at least one external apparatuses, second response information (help request information) is received from one of the at least one external apparatuses,
the information transmitter (wireless transmitter 140) transmits second others' response information (others' help request information) to the other of the at least one external apparatuses.

In the information processing apparatus or the information transmission system having the above configuration, for example, a caregiver who is treating the abnormality of the care-requiring person is able to easily transmit a help request to another caregiver.

Preferably, the information processing apparatus or the information transmission system further includes a terminal state information storage unit (memory 130) that stores terminal state information indicating a state of the external apparatus, wherein
the information transmitter (wireless transmitter 140) inhibits transmission of the state information to the external apparatus whose terminal state information stored in the terminal state information storage unit indicates a predetermined state.

The information processing apparatus or the information transmission system having the above configuration is able to avoid transmitting the state information to the external apparatus in a busy state or in an OFF state, for example. Accordingly, the abnormality information can be transmitted exclusively to the mobile terminals possessed by the caregivers who are able to actually treat the abnormality of the care-requiring person.

Preferably, the information processing apparatus or the information transmission system further includes a terminal identification information storage unit (memory 130) that stores terminal identification information associated with the external apparatus, wherein
when transmitting information to the external apparatus, the information transmitter (wireless transmitter 140) adds the terminal identification information to the information.

The information processing apparatus or the information transmission system having the above configuration enables the caregiver to readily recognize information about exactly which caregiver is treating the abnormality as long as, for example, the names of the caregivers who possess the external apparatuses are preliminarily stored as the terminal identification information in the storage unit. Accordingly, a care system capable of a more appropriate treatment can be provided.

In the information processing apparatus, the information transmission system, or the electronic apparatus, it is preferable that the data is at least one of data detected from photographing data and data based on biometric information.

According to the information processing apparatus, the information transmission system, or the electronic apparatus having the above configuration; a care system, or the like, can be provided that is able to give an appropriate treatment to an abnormality occurring in the detection object.

Preferably, the information processing apparatus further includes:
a state detection unit that detects a state of the detection object; and
a terminal detection unit that detects a state of proximity of the external apparatus, wherein
the information transmitter determines an external apparatus to which the state information is to be transmitted, based on a result of detection performed by the state detection unit and a result of detection performed by the terminal detection unit.

### BRIEF DECRIPTION OF THE DRAWINGS

Fig. 1 is a diagram conceptually showing the whole of a care system;
Fig. 2 shows a specific example of a configuration of a server;
Fig. 3 is a flowchart depicting a detection process performed by a bedside sensor in a specific example;
Fig. 4 is a flowchart depicting a process performed by the server in a specific example;
Fig. 5 shows an exemplary operation of the care system;
Fig. 6 shows an exemplary operation of the care system;
Fig. 7 shows an exemplary operation of the care system;
Fig. 8 shows an exemplary operation of the care system;
Fig. 9 shows an exemplary operation of the care system;
Fig. 10 shows an exemplary display on a mobile terminal according to an embodiment 3;
Fig. 11 shows an exemplary display on the mobile terminal according to the embodiment 3;
Fig. 12 shows an exemplary display on the mobile terminal according to the embodiment 3;
Fig. 13 shows an exemplary display on the mobile terminal according to the embodiment 3;
Fig. 14 shows an exemplary display on the mobile terminal according to the embodiment 3;
Fig. 15 shows an exemplary display on the mobile terminal according to an embodiment 4;
Fig. 16 shows an exemplary display on the mobile terminal according to an embodiment 5;
Fig. 17 shows an exemplary display on the mobile terminal according to the embodiment 5; and
Fig. 18 shows an exemplary display on the mobile terminal according to an embodiment 7.

### DECRIPTION OF EXEMPLARY EMBODIMENTS

With reference to the drawings, a specific description of some embodiments of the present invention will be given based on the following configurations. It should be noted that the embodiments described below are merely illustrative examples of the present invention and not to be construed as limiting the technical scope of the present invention. In the drawings, the same elements are denoted by the same reference signs, and repetitive description may be omitted.
1. Embodiment 1
   (1) Exemplary Configuration of Care System
   (2) Exemplary Operation of Care System
2. Embodiment 2
3. Embodiment 3
4. Embodiment 4
5. Embodiment 5
6. Embodiment 6
7. Embodiment 7
8. Supplementary Remarks

### (1. Embodiment 1)

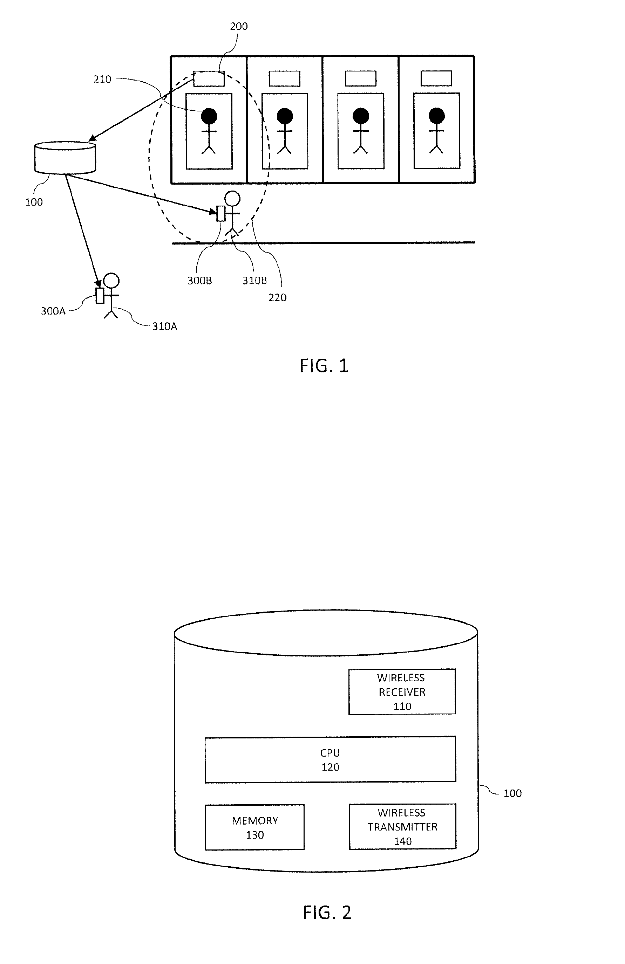

First, an embodiment 1 of the present invention will be described with reference to Figs. 1 to 9.

### ((1) Exemplary Configuration of Care System)

Fig. 1 is a diagram conceptually showing the whole of a care system of this embodiment. As shown in Fig. 1, the care system of this embodiment includes a server 100, a bedside sensor 200, and mobile terminals 300A, 300B. The bedside sensor 200 is installed in or near a bed of a care-requiring person 210. The mobile terminals 300A, 300B are carried by caregivers 310A, 310B, respectively. Each care-requiring person is provided with a corresponding bedside sensor, and each caregiver carries a mobile terminal. In an example shown in Fig. 1, each care-requiring person 210 is in a private room, the caregiver 310A is in a place remote from the care-requiring person 210, and the caregiver 310B is in a hallway near the care-requiring person 210.

### (Bedside Sensor 200)

The bedside sensor 200 functions as: (1) an abnormality detection unit that detects an abnormality of the care-requiring person 210; (2) a terminal detection unit that detects proximity of the mobile terminal 300A, 300B; and (3) a wireless transmitter that wirelessly transmits data to the server 100.

The bedside sensor 200 detects a motion of the care-requiring person 210, and based on a result of the detection, detects an abnormality. The detection of a motion of the care-requiring person 210 is implemented by photographing with a camera. The detection of an abnormality is implemented by image-processing a result of the photographing with the camera and detecting a situation where the care-requiring person 210 is struggling, a situation where no micromotion is observed (supposedly, breathing is stopped), a situation where the care-requiring person 210 moves away from the bed, or the like. Here, the detection of an abnormality of the care-requiring person 210 may not necessarily be implemented with use of the camera, and it can be implemented by detection of biometric information of pulse, body temperature, or the like, of the care-requiring person 210.

The bedside sensor 200 performs wireless communication with the mobile terminal 300A, 300B, to thereby detect the presence of the mobile terminal 300A, 300B within a detectable range 220. The wireless communication between the bedside sensor 200 and the mobile terminal 300A, 300B is allowed only within the detectable range 220.

When the bedside sensor 200 detects an abnormality of the care-requiring person 210 or when the bedside sensor 200 detects proximity of the mobile terminal 300A, 300B; the bedside sensor 200 wirelessly transmits information of the detection to the server 100. Here, wired communication may also be adoptable for the transmission of information from the bedside sensor 200 to the server 100.

### (Detectable Range 220)

The detectable range 220 means a range in which the bedside sensor 200 is able to detect that a mobile terminal is in proximity.

### (Server 100)

Fig. 2 shows a specific example of a configuration of the server 100. As shown in Fig. 2, the server 100 includes a wireless receiver 110, a CPU 120, a memory 130, and a wireless transmitter 140.

### (Wireless Receiver 110)

The wireless receiver 110 receives abnormality detection information about detection of the abnormality of the care-requiring person 210 and proximity detection information about detection of the proximity of the mobile terminal (300A, 300B) that are transmitted from the bedside sensor 200. The wireless receiver 110 outputs the received information to the CPU 120.

### (CPU 120)

The CPU 120 receives input information from the wireless receiver 110. The input information includes the abnormality detection information about the care-requiring person 210 and the proximity detection information about the mobile terminal (300A, 300B) that are received from the wireless receiver 110. Based on the received proximity detection information about the mobile terminal (300A, 300B), the CPU 120 stores into the memory 130 the time when the detection of the mobile terminal was started and the time when the detection was terminated, with respect to each bedside sensor. The time when the mobile terminal is in proximity is determined based on the time that an internal timer of the CPU 120 indicates at the time when a signal of detection of the proximity of the mobile terminal is received from the bedside sensor 200. The time when the detection of a certain mobile terminal was started is determined based on a timing of a state change from a state where the certain mobile terminal was not in proximity (not detected) into a state where the certain mobile terminal was in proximity (detected). The time when the detection of the certain mobile terminal was terminated is determined based on a timing of a state change from a state where the certain mobile terminal was in proximity (detected) into a state where the certain mobile terminal was not in proximity (not detected).

Upon reception of the abnormality detection information about the care-requiring person 210, the CPU 120 identifies to which mobile terminal the CPU 120 should transmit abnormality information indicating occurrence of the abnormality. Then, the CPU 120 transmits the abnormality information to the identified mobile terminal via the wireless transmitter 140. A specific description of these operations including the process performed by the CPU 120 will be given later.

### (Memory 130)

The memory 130 stores, with respect to each bedside sensor, the time when the detection of the mobile terminal was started and the time when the detection of the mobile terminal was terminated that are determined based on the proximity detection information about the mobile terminal received from the CPU 120. The memory 130 is able to store not only the above-mentioned information but also various types of information.

### (Wireless Transmitter 140)

Based on a command from the CPU 120, the wireless transmitter 140 wirelessly transmits the abnormality information to a predetermined mobile terminal (300A, 300B). Here, the wireless transmitter 140 may be combined with the wireless receiver 110 and be a transmitter-receiver.

### (Mobile Terminal 300A, 300B)

Each of the mobile terminals 300A and 300B is a terminal capable of wireless communication with the server 100 and the bedside sensor 200. Each caregiver possesses one mobile terminal. The mobile terminal 300A, 300B may be an electronic terminal or electronic apparatus including a cellular phone, a smart phone, or the like. Each of the mobile terminals 300A and 300B has terminal's unique identification information. The server 100, the bedside sensor 200, and other devices are able to identify the mobile terminal based on the terminal's unique identification information. Each of the mobile terminals 300A and 300B includes a communication unit that performs wireless communication with the server 100 and the bedside sensor 200. The communication unit may be a receiver adapted only for reception of information. Each of the mobile terminals 300A and 300B also includes a touch panel that displays an image based on received information and that detects an operation performed by a user. The touch panel includes a display unit implemented by a liquid crystal display (LCD), an organic electroluminescent display, or the like. Alternatively, the touch panel may be a display unit having a function of displaying an image.

### ((2) Exemplary Operation of Care System)

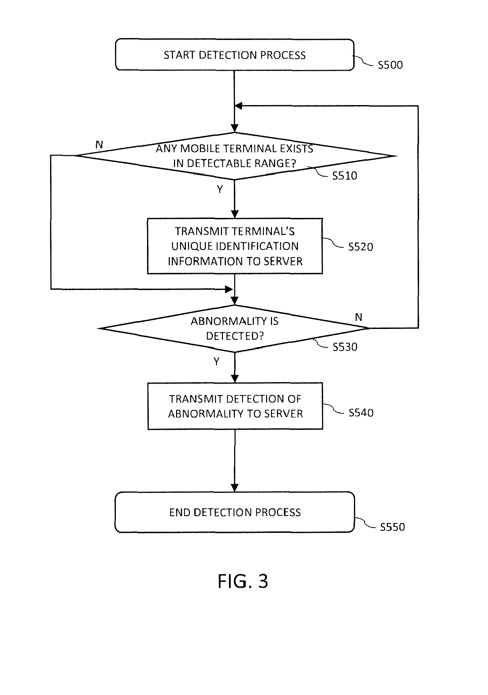

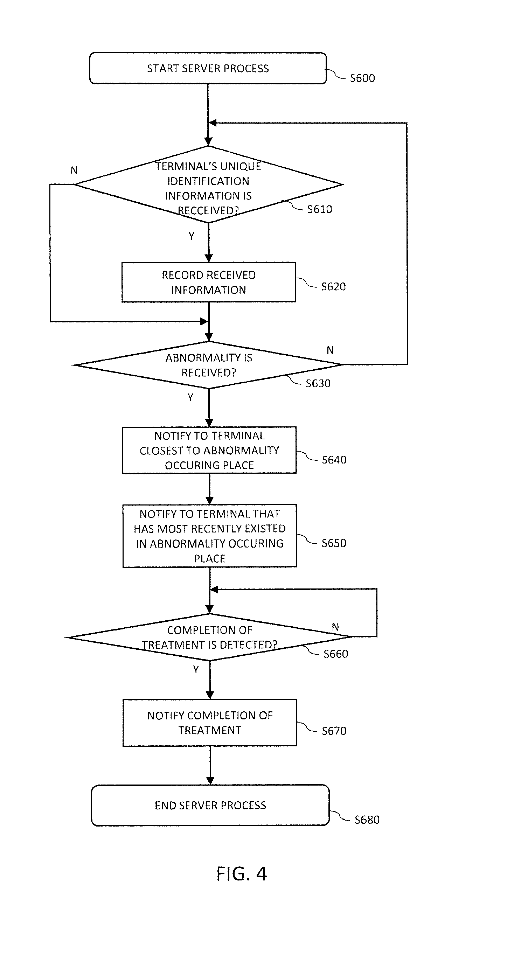

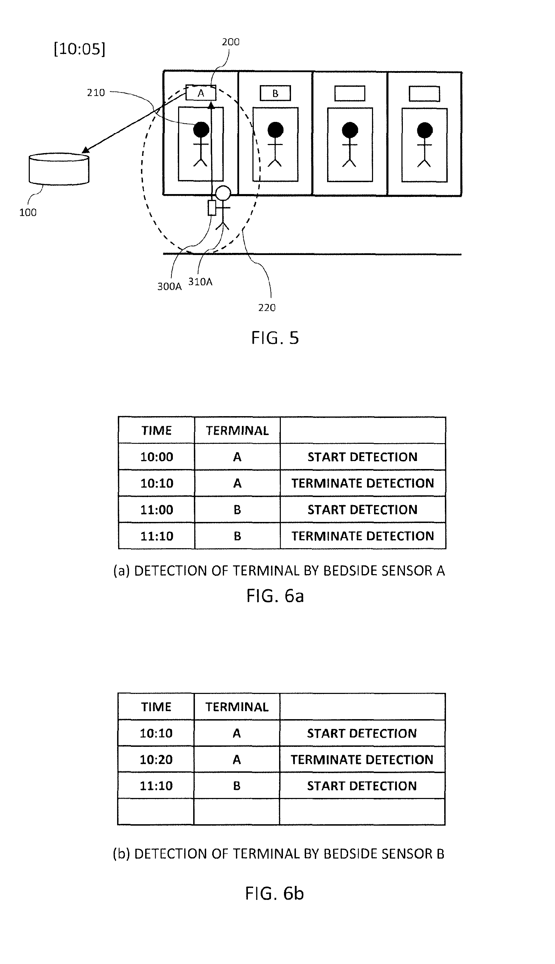

Next, an exemplary operation of the care system of this embodiment will be described with reference to Figs. 3 to 9. Firstly, referring to Figs. 3 and 4, a flow of a process performed in the bedside sensor 200 and the server 100 will be described, and then referring to Figs. 5 to 9, a more specific example of the operation will be described.

### (S500 to S510)

Fig. 3 is a flowchart depicting a detection process performed by the bedside sensor 200 in a specific example. When a detection process is started (S500), the bedside sensor 200 firstly checks whether or not there is any mobile terminal existing in the detectable range 220 (S510). If no mobile terminal exists in the detectable range 220 (S510:N), the process goes to S530.

### (S520)

If any mobile terminal exists in the detectable range 220 (S510:Y), the bedside sensor 200 transmits the terminal's unique identification information of the detected mobile terminal to the server 100. The server 100 receiving this information performs a process which will be described later.

### (S530)

Then, the bedside sensor 200 checks whether or not any abnormality occurs in the care-requiring person 210 (S530). More specifically, the bedside sensor 200 photographs the care-requiring person 210 with the camera, to check whether or not a situation is detected where the care-requiring person 210 is struggling, where no micromotion is observed (supposedly, breathing is stopped), or where the care-requiring person 210 moves away from the bed, or the like. If no abnormality occurs in the care-requiring person 210 (S530:N), the process returns to S510.

### (S540)

If any abnormality occurs in the care-requiring person (S530:Y), the bedside sensor 200 transmits abnormality detection information to the server 100 (S540), and the detection process ends. Thereafter, the bedside sensor 200 repeatedly performs the detection process.

### (S600)

Fig. 4 is a flowchart depicting a process performed by the server 100 in a specific example. When a server process is started (S600), the wireless receiver 110 of the server 100 outputs, to the CPU 120, the information received from the bedside sensor 200, namely, the terminal's unique identification information (see S520) and the abnormality detection information about the care-requiring person 210 (see S540). The terminal's unique identification information is proximity detection information about the mobile terminal.

### (S610 to S620)

If the CPU 120 receives the terminal's unique identification information of the mobile terminal (S610:Y); based on the information, the CPU 120 stores into the memory 130 the time when the detection of the mobile terminal (that is identified based on the terminal's unique identification information) was started and the time when the detection was terminated with respect to each bedside sensor. The time when the detection of the mobile terminal was started and the time when the detection was terminated are determined with use of the internal timer of the CPU 120.

### (S630)

If the CPU 120 does not receive the terminal's unique identification information of the mobile terminal (S610:N) or after the CPU 120 stores the received information into the memory 130 (after S620), the CPU 120 checks whether or not the abnormality detection information about the care-requiring person 210 is received from the bedside sensor 200 (S630). If the abnormality detection information about the care-requiring person 210 is not received (S630:N), the CPU 120 repeatedly executes the process of S610.

### (S640)

If the abnormality detection information about the care-requiring person 210 is received (S630:Y), the CPU 120 identifies the bedside sensor 200 having issued this abnormality detection information as an abnormality occurring place. The CPU 120 then identifies a mobile terminal closest to the abnormality occurring place, and issues the abnormality information to the identified mobile terminal via the wireless transmitter 140 (S640). This identification of the mobile terminal closest to the abnormality occurring place by the CPU 120 is based on mobile terminal detection information a stored in the memory 130.

### (S650)

Then, the CPU 120 identifies a mobile terminal that has been present in (proximal to) the abnormality occurring place immediately before occurrence of the abnormality, and transmits the abnormality information to the identified mobile terminal via the wireless transmitter 140 (S650). This identification of the mobile terminal by the CPU 120 is based on the mobile terminal detection information stored in the memory 130. The mobile terminal identified at this time, to which the abnormality information is to be transmitted, is different from the aforementioned mobile terminal closest to the abnormality occurring place. In this manner, the CPU 120 issues the abnormality information to two mobile terminals.

### (S660 to S670)

The CPU 120 waits for detection of completion of a treatment (S660). If completion of the treatment is detected, the CPU 120 transmits information indicating completion of the treatment to the mobile terminal (S670). The detection of completion of the treatment is determined based on a situation where, after the CPU 120 transmits the aforementioned abnormality information, the bedside sensor 200 located in the abnormality occurring place detects proximity of one of the two mobile terminals having received the abnormality information and then this mobile terminal stops being detected. That is, it is estimated that the treatment for treating the abnormality of the care-requiring person is completed based on a result of detection of a situation where, after the server 100 transmits the abnormality information, a caregiver approaches the care-requiring person and then leaves the care-requiring person.

### (S680)

After the above-described process ends, the server 100 terminates the process (S680).

### (Specific Example of Operation)

A specific example of an operation will be described with reference to Figs. 5 to 9.

Fig. 5 shows a situation where the caregiver 310A possessing the mobile terminal 300A is near the care-requiring person 210 (at 10:05). At this time, the bedside sensor 200 detects that the mobile terminal 300A exists in the detectable range 220 (S510), and transmits the terminal's unique identification information to the server 100 (S520).

Fig. 6 shows the time when detection of the mobile terminal was started and the time when the detection was terminated with respect to each bedside sensor, which are determined based on the information (terminal's unique identification information) of detection of the proximity of the mobile terminals stored in the memory 130 (see S610 and S620). Referring to the uppermost in Fig. 6(a), the information about the proximity of the mobile terminal 300A to the bedside sensor A (200) is stored. It is indicated that the mobile terminal A (300A) starts being detected by the bedside sensor A (200) at time of 10:00.

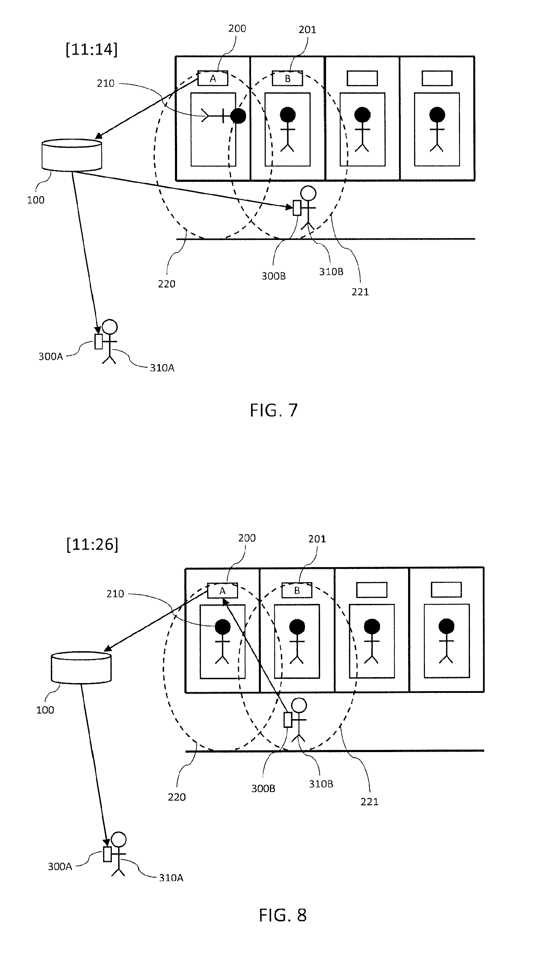

Fig. 7 shows a situation where an abnormality occurs in the care-requiring person 210 (at 11:14). The caregiver 310A possessing the mobile terminal 300A is in a place remote from the care-requiring person 210, and the caregiver 310B possessing the mobile terminal 300B is in a detectable range 221 of a bedside sensor B (201). In this situation, the information stored in the memory 130 indicating the time when detection of the mobile terminal was started and the time when the detection was terminated for each bedside sensor is as shown in Fig. 6.

Referring to Fig. 6, the bedside sensor A (200): (1) at 10:00, started detecting the mobile terminal 300A; (2) at 10:10, terminated the detection of the mobile terminal 300A; (3) at 11:00, started detecting the mobile terminal 300B; and (4) at 11:10, terminated the detection of the mobile terminal 300B. This indicates that the caregiver 310A possessing the mobile terminal 300A was near the bedside sensor A (200) in a time period from 10:00 to 10:10, and that the caregiver 310B possessing the mobile terminal 300B was near the bedside sensor A (200) in a time period from 11:00 to 11:10. On the other hand, the bedside sensor B (201): (1) at 10:10, started detecting the mobile terminal 300A; (2) at 10:20, terminated the detection of the mobile terminal 300A; and (3) at 11:10, started detecting the mobile terminal 300B. This indicates that the caregiver 310A possessing the mobile terminal 300A was near the bedside sensor B (201) in a time period from 10:10 to 10:20, and that the caregiver 310B possessing the mobile terminal 300B was near the bedside sensor B (201) at the present time (11:14).

Upon detecting the abnormality of the care-requiring person 210, the bedside sensor A (200) transmits abnormality detection information to the server 100 (S540). The server 100 receives the abnormality detection information (S630). In response to this, based on the information stored in the memory 130, the CPU 120 of the server 100 transmits the abnormality information to the mobile terminal 300B closest to the abnormality occurring place (see S640) and to the mobile terminal 300A that has existed in the abnormality occurring place just before the mobile terminal 300B (see S650). The caregivers 310A and 310B see the mobile terminals receiving the abnormality information, and go to the care-requiring person 210 having the abnormality in order to treat the care-requiring person 210.

The caregiver 310B immediately arrives at the care-requiring person 210, and completes a treatment of the abnormality of the care-requiring person 210 in a short time. Then, the caregiver 310B leaves the care-requiring person 210. At this time, the caregiver 310A does not yet arrive at the care-requiring person 210.

Fig. 8 shows the above-described situation (at 11:26). The caregiver 310B possessing the mobile terminal 300B goes out of the detectable range 220 of the bedside sensor A (200), and enters the detectable range 221 of the bedside sensor B (201).

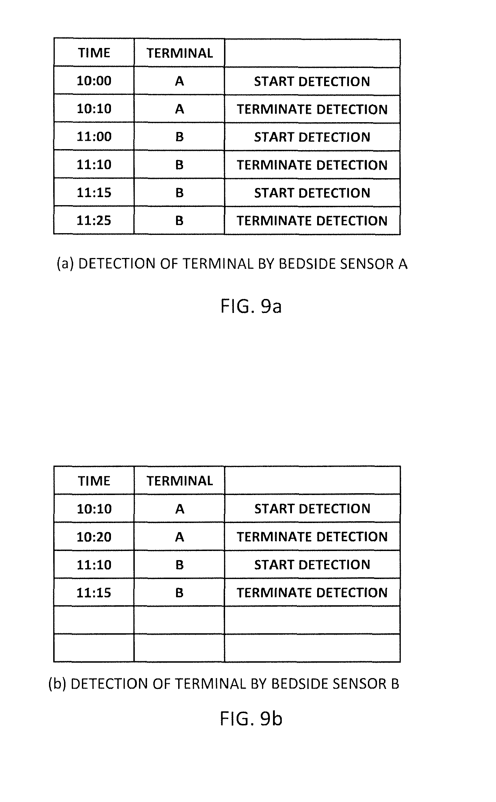

Fig. 9 shows information indicating the time when detection of the mobile terminal was started and the time when the detection was terminated with respect to each bedside sensor (see S610 and S620), which are stored in the memory 130 at this time. The caregiver 310B, who saw the mobile terminal 300B receiving the abnormality information at time of 11:14 at which the abnormality occurred in the care-requiring person 210, immediately went to the care-requiring person 210. Therefore, the mobile terminal detection information for the bedside sensor B (201) contains information indicating that the detection of the mobile terminal 300B was terminated at 11:15. The mobile terminal detection information for the bedside sensor A (201) contains information indicating that the mobile terminal 300B was detected in a time period from 11:15 to 11:25. From this, the CPU 120 determines that the caregiver 310B who arrived at the care-requiring person 210 at 11:15 completed a treatment of the abnormality and left the care-requiring person 210 at 11:25.

As described above, the CPU 120 detects completion of the treatment of the abnormality (S660), and transmits, to the mobile terminal 300A, treatment completion information that indicates completion of the treatment (S670) (see Fig. 8).

Although the abnormality information is transmitted to the two mobile terminals 300A and 300B in the above-described process, it may be acceptable that the abnormality information is transmitted to either one of the two mobile terminals 300A and 300B alone. It is however preferable that the abnormality information is transmitted to both of the mobile terminals 300A and 300B, because it enables the abnormality of the care-requiring person 210 to be treated appropriately.

### (2. Embodiment 2)

Next, an embodiment 2 of the present invention will be described. This embodiment is different from the embodiment 1 in that, instead of the step of transmitting the abnormality information to the mobile terminal 300A (see S650), the abnormality information is transmitted to a mobile terminal 300C (not shown) possessed by a caregiver who is responsible for the care-requiring person 210. The mobile terminal 300C has the same configuration as the configuration of the mobile terminal 300A or 300B.

The memory 130 stores, as mobile terminal information, information of caregivers each associated with each care-requiring person. The mobile terminal information associated with each caregiver is preliminarily registered by a user.

Based on the above-described information, instead of the process of S650 shown in Fig. 4, the server 100 transmits the abnormality information to the mobile terminal 300C possessed by the caregiver who is responsible for the care-requiring person having the abnormality, which is preliminarily registered (see S650).

In the example given above, the abnormality information is transmitted to the mobile terminal 300B and to the mobile terminal 300C possessed by the responsible caregiver. Alternatively, the abnormality information may be transmitted to the mobile terminal 300A and to the mobile terminal 300C possessed by the responsible caregiver. It is however more preferable that the abnormality information is transmitted to the mobile terminal 300B and to a mobile terminal possessed by a responsible caregiver, because a response to an emergency and an appropriate treatment for the care-requiring person 210 can be achieved concurrently. It may be also acceptable that this embodiment and the embodiment 1 are combined such that the abnormality information is transmitted to three mobile terminals of the mobile terminal 300A, the mobile terminal 300B, and the mobile terminal 300C possessed by the responsible caregiver. This configuration enables the abnormality of the care-requiring person 210 to be treated by three persons.

Adoption of the information processing apparatus or the information transmission system described in the embodiment 1 or 2 enables occurrence of an abnormality to be notified to two or more persons from: the caregiver B (310B) who possesses the mobile terminal (300B) and exists near the care-requiring person (210) having the abnormality; the caregiver A (310A) who possesses the mobile terminal (300A) and existed near the care-requiring person immediately before occurrence of the abnormality; and the caregiver who is responsible for the care-requiring person (210) having the abnormality. As a result, the caregiver B is able to respond to an emergency, while the caregiver A who is likely to recognize the condition of the care-requiring person and the responsible caregiver who is likely to familiar with a history of the care-requiring person are able to give an appropriate treatment.

The server 100 transmits the treatment completion information to the mobile terminal based on the state of proximity of the mobile terminal. Accordingly, a caregiver who does not know the status of the treatment of the abnormality can be automatically notified of completion of the treatment of the abnormality of the care-requiring person.

The server 100 includes the memory 130 that stores a result of detection performed by the terminal detection unit and a detection time associated with the result of detection. This makes it possible that the server 100 stores the status of detection of an electronic terminal (the mobile terminal 300A or 300B) detected by the bedside sensor 200. Accordingly, a process of notifying occurrence of an abnormality can be readily executed.

### (3. Embodiment 3)

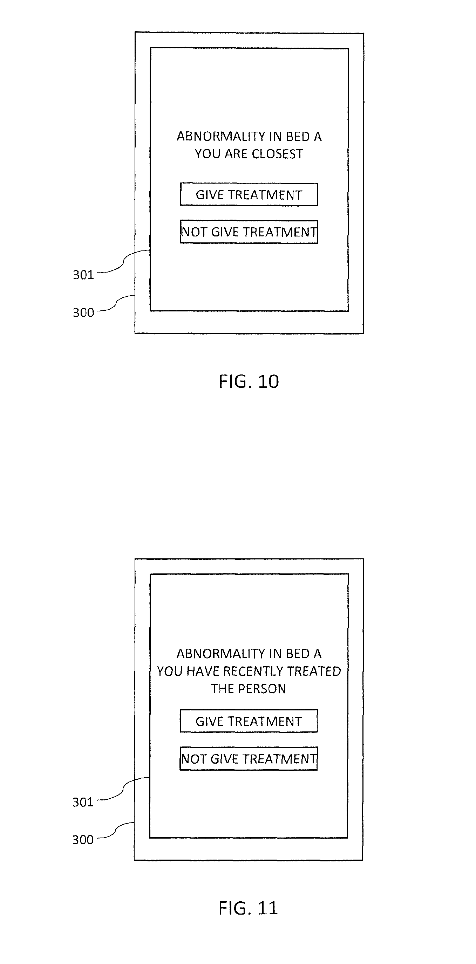

Next, an embodiment 3 of the present invention will be described with reference to Figs. 10 to 14. This embodiment is different from the embodiments 1 and 2 in that the mobile terminal has a function of transmitting, to other mobile terminals, treatment declaration information that declares engagement in the treatment of the abnormality. A specific description is as follows.

The mobile terminal 300 (300A, 300B, 300C) includes a touch panel 301 that displays an image and detects an operation performed by a user. Upon reception of the abnormality information from the server 100, the mobile terminals 300 (300A, 300B, 300C) displays on the touch panel 301 a display for selecting whether or not to treat the abnormality. The abnormality information contains information indicating whether the mobile terminal having received this abnormality information is (1) the mobile terminal (300B) closest to the abnormality occurring place, (2) the mobile terminal (300A) having existed near the abnormality just before the mobile terminal (300B), or (3) the mobile terminal (300C) possessed by the caregiver who is responsible for the care-requiring person having the abnormality. Contents according to this information are displayed on the touch panel 301.

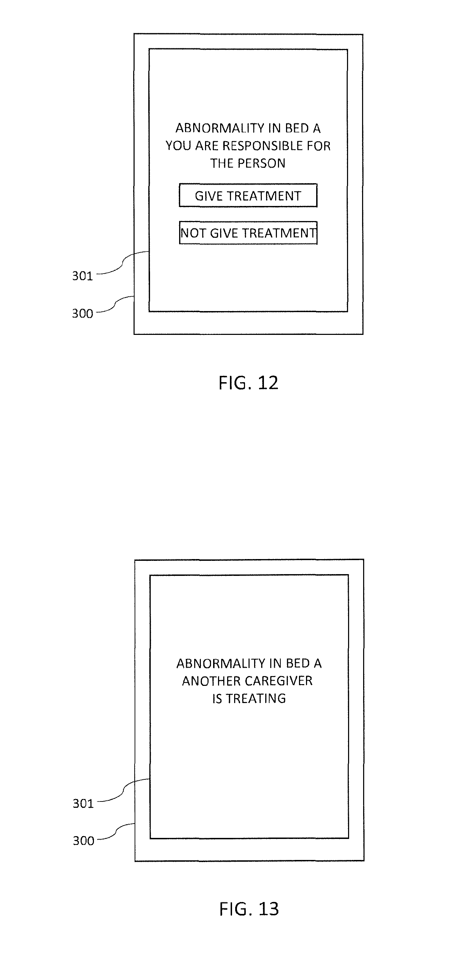

Figs. 10 to 12 show display states of the touch panel 301 of the mobile terminal 300 at this time. Fig. 10 shows a display state of (1) the mobile terminal (300B) closest to the abnormality occurring place. Fig. 11 shows a display state of (2) the mobile terminal (300A) having existed near the abnormality just before the mobile terminal (300B) that is currently closest to the abnormality occurring place. Fig. 12 shows a display state of (3) the mobile terminal (300C) possessed by the caregiver who is responsible for the care-requiring person having the abnormality. If the caregiver possessing the mobile terminal 300 performs an operation representing "give treatment", the mobile terminal 300 transmits the treatment declaration information to the server 100. If the caregiver possessing the mobile terminal 300 performs an operation representing "not give treatment", the mobile terminal 300 transmits non-treatment declaration information to the server 100. Each of the treatment declaration information and the non-treatment declaration information contains terminal's unique identification information that allows identification of the mobile terminal 300 having transmitted the treatment declaration information or the non-treatment declaration information.

The wireless receiver 110 of the server 100 receives the treatment declaration information or the non-treatment declaration information from the mobile terminal 300A, 300B, or 300C, and outputs the received treatment declaration information or non-treatment declaration information to the CPU 120.

Upon reception of the treatment declaration information, the CPU 120 identifies the mobile terminal having transmitted this treatment declaration information based on the terminal's unique identification information contained in this treatment declaration information. The CPU 120 then controls the wireless transmitter 140 so as to transmit others' treatment declaration information to the mobile terminal that is different from the identified mobile terminal and that has received the abnormality information simultaneously with the identified mobile terminal. The mobile terminal 300 having received the others' treatment declaration information causes the touch panel 301 thereof to display indication that another caregiver is treating the occurring abnormality. Fig. 13 shows a display state of the touch panel 301 of the mobile terminal 300 at this time.

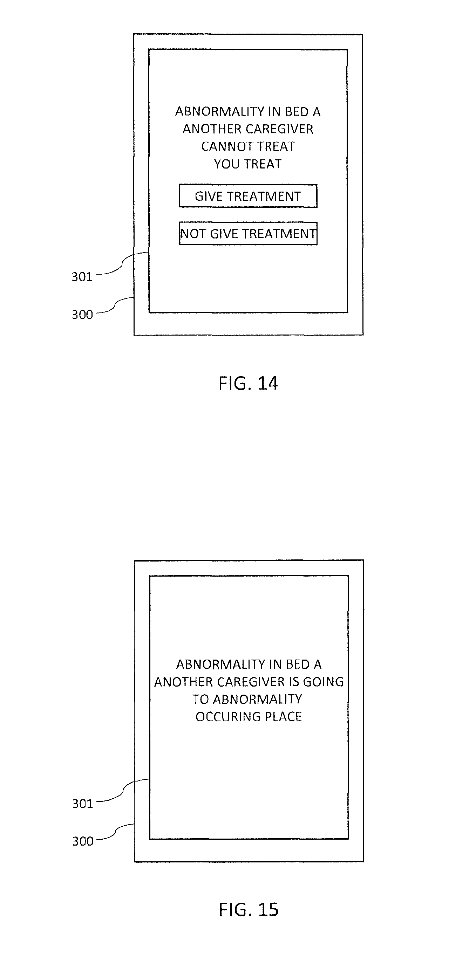

Upon reception of the non-treatment declaration information, the CPU 120 identifies the mobile terminal having transmitted this non-treatment declaration information based on the terminal's unique identification information contained in this non-treatment declaration information. The CPU 120 then transmits others' non-treatment declaration information to the mobile terminal that is different from the identified mobile terminal and that has received the abnormality information simultaneously with the identified mobile terminal. The mobile terminal 300 having received the others' non-treatment declaration information displays thereon indication that another caregiver cannot treat the occurring abnormality. Fig. 14 shows a display state of the touch panel 301 of the mobile terminal 300 at this time.

If all of the caregivers who possess the mobile terminals having received the abnormality information perform the operation representing "not give treatment", the CPU 120 receives the non-treatment declaration information from all of the mobile terminals that have received the abnormality information. In this case, the CPU 120 transmits the abnormality information to a mobile terminal other than the mobile terminals that have already received the abnormality information.

Adoption of the information processing apparatus or the information transmission system having the above-described configuration enables a certain caregiver who possesses the mobile terminal to easily notify caregivers who possesses other mobile terminals that the certain caregiver is going to treat the abnormality. In a case where none of the caregivers can treat the abnormality, another caregiver can be notified of it.

### (4. Embodiment 4)

Next, an embodiment 4 of the present invention will be described with reference to Fig. 15. This embodiment is different from the embodiments 1 and 2 in that a situation where the mobile terminal having received the abnormality information is approaching the abnormality occurring place is detected, and a result of the detection is transmitted to other mobile terminals. A specific description is as follows.

In the embodiment 1 described above, based on a result of detection of the situation where, after the server 100 transmits the abnormality information, a caregiver approaches the care-requiring person and then leaves the care-requiring person, it is estimated that the treatment for treating the abnormality of the care-requiring person is completed, and information indicating it is transmitted to the mobile terminals (see S660 and S670). In this embodiment, at a time point when the caregiver A approaches the care-requiring person after the server 100 transmits the abnormality information, others' proximity information is transmitted to the caregiver B. The others' proximity information indicates that another caregiver A is approaching the care-requiring person.

To be more specific, if, after the abnormality information is transmitted from the wireless transmitter 140 of the server 100, the bedside sensor 200 located in the abnormality occurring place detects that any of the mobile terminals having received the abnormality information is in proximity; the bedside sensor 200 transmits a result of this detection to the server 100. Based on the result of this detection, the CPU 120 of the server 100 transmits the others' proximity information to other mobile terminals 300 that have received the abnormality information.

Upon reception of the others' proximity information, the mobile terminal 300 causes the touch panel 301 thereof to display information indicating that another caregiver is in proximity to the abnormality occurring place. Fig. 15 shows a display state of the touch panel 301 of the mobile terminal 300 at this time.

Adoption of the information processing apparatus or the information transmission system having the above-described configuration makes it easy to notify that a certain caregiver who possesses the mobile terminal is approaching the care-requiring person having the abnormality, to caregivers who possess other mobile terminals. Accordingly, the caregivers who possess other mobile terminals can readily know that another caregiver is going to treat the abnormality.

### (5. Embodiment 5)

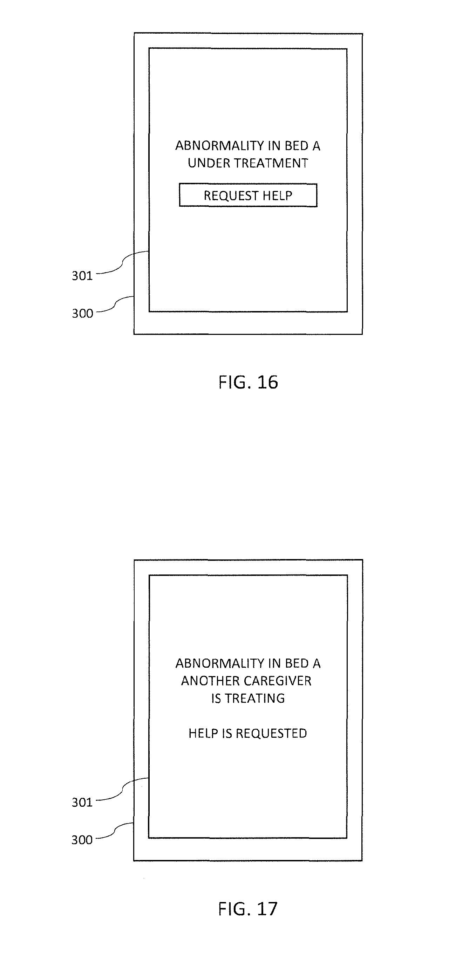

Next, an embodiment 5 of the present invention will be described with reference to Figs. 16 and 17. This embodiment is different from the above-described embodiments in that the caregiver who is currently treating the abnormality occurring in the care-requiring person is able to issue a help request to other caregivers. A specific description is as follows.

Fig. 16 shows a display state of the touch panel 301 of the mobile terminal 300 possessed by the caregiver who is currently treating the abnormality. Various methods may be used to identify a situation where a caregiver who possesses a certain mobile terminal 300 is currently treating the abnormality. For example, such a situation can be identified based on the fact that the treatment declaration information is transmitted from the certain mobile terminal 300.

If the caregiver performs an operation for requesting a help, the mobile terminal 300 transmits help request information to the server 100. The help request information contains the terminal's unique identification information that allows identification of the mobile terminal 300 having transmitted the help request information.

Upon reception of the help request information from the mobile terminal 300, the wireless receiver 110 of the server 100 outputs the help request information to the CPU 120.

Upon reception of the help request information, the CPU 120 identifies the mobile terminal having transmitted this help request information based on the terminal's unique identification information contained in this help request information. The CPU 120 then controls the wireless transmitter 140 so as to transmit others' help request information to the mobile terminal that is different from the identified mobile terminal and that has received the abnormality information simultaneously with the identified mobile terminal. The CPU 120 may transmit the others' help request information to all mobile terminals except the mobile terminal that has transmitted the help request information. Alternatively, when the help request information is transmitted, the CPU 120 may transmit the others' help request information to a mobile terminal located near an abnormality occurring place. The mobile terminal 300 having received the others' help request information causes the touch panel 301 thereof to display indication that another caregiver who is treating the occurring abnormality is requesting a help. Fig. 17 shows a display state of the touch panel 301 of the mobile terminal 300 at this time.

Adoption of the information processing apparatus or the information transmission system having the above-described configuration enables a caregiver who is treating the abnormality of the care-requiring person to easily transmit a help request to other caregivers.

### (6. Embodiment 6)

Next, an embodiment 6 of the present invention will be described. This embodiment is different from the above-described embodiments in that the server 100 stores the state of each mobile terminal and executes a process of avoid transmitting the abnormality information to a mobile terminal in a predetermined state. A specific description is as follows.

The state of each mobile terminal is stored in the memory 130 of the server 100. The state of the mobile terminal means the state of a caregiver who possesses the mobile terminal. Examples thereof include: a busy state which indicates a state where a caregiver is engaged in another task so that he/she cannot treat the abnormality of the care-requiring person; an OFF state (or resting state) which indicates a state where a caregiver is taking a meal break or the like so that he/she cannot immediately treat the abnormality of the care-requiring person; and an active state which indicates a state where a caregiver is able to treat the abnormality of the care-requiring person.

When transmitting the abnormality information, the CPU 120 of the server 100 checks the state of each mobile terminal, which is stored in the memory 130. Then, the CPU 120 performs such a control as to transmit the abnormality information only to the mobile terminal that is able to treat the abnormality.

Adoption of the information processing apparatus or the information transmission system having the above-described configuration is able to avoid transmitting the abnormality information to a mobile terminal in the busy state or in the OFF state, for example Accordingly, the abnormality information can be transmitted exclusively to the mobile terminals possessed by the caregivers who are able to treat the abnormality of the care-requiring person.

### (7. Embodiment 7)

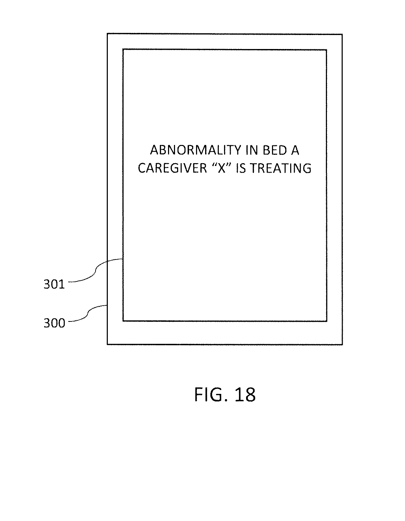

Next, an embodiment 7 of the present invention will be described with reference to Fig. 18. This embodiment is different from the above-described embodiments in that terminal identification information associated with each mobile terminal is preliminarily registered in the memory 130 of the server 100 and thereby, for example, the name of a caregiver who possesses the mobile terminal is displayed on the mobile terminal. A specific description is as follows.

In the memory 130, a table of the terminal identification information is preliminarily registered. The table associates a mobile terminal with the name of a caregiver who possesses the mobile terminal. Therefore, for example, on the touch panel 301 of the mobile terminal 300 having received the others' treatment declaration information, information indicating exactly who is treating is displayed. Fig. 18 shows a display state of the touch panel 301 of the mobile terminal 300 at this time.

To be more specific, when transmitting the others' treatment declaration information, the others' non-treatment declaration information, the others' help request information, or the others' proximity information to the mobile terminal 300, the CPU 120 adds, to this information, the terminal identification information indicating the name of a caregiver associated with the mobile terminal having transmitted the corresponding treatment declaration information, the mobile terminal having transmitted the corresponding non-treatment declaration information, the mobile terminal having transmitted the help request information, or the mobile terminal that is approaching the abnormality occurring place. Accordingly, the mobile terminal 300 having received the others' treatment declaration information, the others' non-treatment declaration information, the others' help request information, or the others' proximity information is able to display, on the touch panel 301 thereof, information about who treats the abnormality based on the terminal identification information.

The terminal identification information may be not only the name of the caregiver but also an employee code, a nickname, or the like, as long as it allows identification of the caregiver.

Adoption of the information processing apparatus or the information transmission system having the above-described configuration enables the caregiver to readily recognize information about exactly which caregiver is treating the abnormality as long as, for example, the names of the caregivers who possess the mobile terminals are preliminarily stored as the terminal identification information in a storage unit. Accordingly, a care system capable of a more appropriate treatment can be provided.

### (8. Supplementary Remarks)

Hereinbefore, specific descriptions of the embodiments of the present invention have been given. These embodiments are merely illustrative. The scope of the present invention is not restricted to the illustrative embodiments. The present invention should be construed as widely as those skilled in the art can appreciate.

Although the above-described embodiments detect an abnormality with the bedside sensor 200, the detection of an abnormality may be performed by the server 100. In such a case, the bedside sensor 200 transmits, for example, information obtained by photographing with the camera to the server 100, and the CPU 120 of the server 100 detects occurrence of the abnormality based on the information.

The above-described embodiments illustrate the example case where the bedside sensor 200 functions as the abnormality detection unit, the terminal detection unit, and the wireless transmitter; however, it may not always be necessary that all of these functions are provided in the bedside sensor 200. All or part of the functions may be provided separately from the bedside sensor 200. That is, these functions may be provided separately from the bed.

The detection of the mobile terminal may be performed by an element different from the terminal detection unit of the bedside sensor 200. For example, a terminal detector may be arranged in a place different from the bed.

The above-described embodiments illustrate the example case where the time when the mobile terminal was detected is fixed based on the internal timer included in the CPU 120 of the server 100; however, the time when the mobile terminal was detected may be fixed based on a timer included in the bedside sensor 200. It is however preferable that the time when the mobile terminal was detected in the server 100 side, because fixing the times by the respective bedside sensors 200 may cause a time lag between the bedside sensors.

The server 100 detects completion of the treatment (S660). This detection may not be automatic detection by the CPU 120, but instead may be based on information inputted by the caregiver who has treated the abnormality of the care-requiring person 210. Such a configuration enables more accurate determination of the fact that the treatment of the abnormality of the care-requiring person 210 is completed. It is however preferable that the CPU 120 automatically detects the completion of the treatment of the abnormality, because it can save the labor of the input by the care-requiring person.

The mobile terminal may be replaced by an electronic apparatus that communicates with the server 100 by wire communication.

The present invention may be suitable for application to a care system.

## Claims

1. An information processing apparatus (100) comprising:
a receiver (110) that receives data indicating a state of a detection object detected by a state detection unit (200); and
an information transmitter (140) that transmits state information to an external apparatus (300A, 300B, and/or 300C) based on the data,
the information transmitter transmitting the state information to at least selected one of:
a first external apparatus (300B) determined to be at a short distance from the detection object;
a second external apparatus (300A) determined to have been in proximity to the detection object; and
a third external apparatus (300C) that is preliminarily determined.

2. The information processing apparatus (100) according to claim 1, wherein
the first external apparatus (300B) is determined based on the detection of the state of the detection object,
the second external apparatus (300A) is determined based on a situation where the second external apparatus has been in proximity to the detection object prior to the first external apparatus (300B).

3. The information processing apparatus (100) according to claim 1 or 2, wherein
the information transmitter (140) transmits the state information to the third external apparatus (300C) and to at least one of the first and second external apparatuses (300B and 300A).

4. The information processing apparatus (100) according to any one of claims 1 to 3, wherein
the first and second external apparatuses (300B and 300A) to which the state information is to be transmitted are determined based on an input received from a terminal detection unit (200) that detects a state of proximity of an external apparatus.

5. The information processing apparatus (100) according to claim 4, wherein
in a case where, after the state information is transmitted to the at least one external apparatuses, the terminal detection unit (200) detects proximity of one of the at least one external apparatuses and then the proximity becomes undetectable,
the information transmitter (140) transmits completion information to the other of the at least one external apparatuses.

6. The information processing apparatus (100) according to claim 4 or 5, further comprising a detection terminal storage unit (130) that stores a result of detection performed by the terminal detection unit (200) and a detection time associated with the result of detection.

7. The information processing apparatus (100) according to any one of claims 4 to 6, wherein
in a case where, after the state information is transmitted to the at least one external apparatuses, first response information is received from one of the at least one external apparatuses,
the information transmitter (140) transmits first others' response information to the other of the at least one external apparatuses.

8. The information processing apparatus (100) according to any one of claims 4 to 7, wherein
in a case where, after the state information is transmitted to the at least one external apparatuses, proximity of one of the at least one external apparatuses is detected,
the information transmitter (140) transmits others' proximity information to the other of the at least one external apparatuses.

9. The information processing apparatus (100) according to any one of claims 4 to 8, wherein
in a case where, after the state information is transmitted to the at least one external apparatuses, second response information is received from one of the at least one external apparatuses,
the information transmitter (140) transmits second others' response information to the other of the at least one external apparatuses.

10. The information processing apparatus (100) according to any one of claims 3 to 9, further comprising a terminal state information storage unit (130) that stores terminal state information indicating a state of the external apparatus, wherein
the information transmitter (140) inhibits transmission of the state information to the external apparatus whose terminal state information stored in the terminal state information storage unit indicates a predetermined state.

11. The information processing apparatus (100) according to any one of claims 4 to 10, further comprising a terminal identification information storage unit (130) that stores terminal identification information associated with the external apparatus, wherein
when transmitting information to the external apparatus, the information transmitter (140) adds the terminal identification information to the information.

12. The information processing apparatus according to any one of claims 1 to 11, wherein
the data is at least one of data detected from photographing data and data based on biometric information.

13. The information processing apparatus according to any one of claims 1 to 12, further comprising:
a state detection unit (200) that detects a state of the detection object; and
a terminal detection unit (200) that detects a state of proximity of the external apparatus, wherein
the information transmitter (140) determines an external apparatus to which the state information is to be transmitted, based on a result of detection performed by the state detection unit and a result of detection performed by the terminal detection unit.

14. The information processing apparatus according to any one of claims 1 to 13, wherein
the third external apparatus is preliminary associated with the detection object.

15. An electronic apparatus comprising:
a receiver that receives a state information transmitted based on data about a state of a detection object; and
a display unit that displays a predetermined image based on the state information,
the receiver receiving the state information that is transmitted to at least selected one of:
a first external apparatus determined to be at a short distance from the detection object;
a second external apparatus determined to have been in proximity to the detection object; and
a third external apparatus that is preliminarily determined.

16. A method for transmitting information, comprising:
a first step of detecting a state of a detection object;
a second step of detecting a state of proximity of an external apparatus; and
a third step of, based on results of detection performed in the first and second steps, transmitting state information to at least selected one of:
a first external apparatus (300B) determined to be at a short distance from the detection object;
a second external apparatus (300A) determined to have been in proximity to the detection object; and
a third external apparatus (300C) that is preliminarily determined.
